# EUROPÄISCHE PATENTSCHRIFT

(11) **EP 1 771 433 B1**
(45) Veröffentlichungstag und Bekanntmachung des Hinweises auf die Patenterteilung: **12.03.2008**
(21) Anmeldenummer: 05762093.2
(22) Anmeldetag: 12.07.2005
(51) Int. Cl.: C07D 311/86

(54) **VERFAHREN ZUR ISOLIERUNG VON ALPHA-MANGOSTIN**
METHOD FOR THE ISOLATION OF ALPHA-MANGOSTIN
PROCEDE POUR ISOLER DE L'ALPHA-MANGOSTIN

(30) Priorität: 17.07.2004 DE 102004034683
(43) Veröffentlichungstag der Anmeldung: 11.04.2007
(73) Patentinhaber: Boehringer Ingelheim Pharma GmbH & Co. KG, 55216 Ingelheim (DE)
(72) Erfinder: SOBOTTA, Rainer, 55218 INGELHEIM (DE); IGNATOW, Hans-Peter, 55218 INGELHEIM (DE)
(74) Vertreter: Hammann, Heinz
(86) Internationale Anmeldenummer: PCT/EP2005/007534
(87) Internationale Veröffentlichungsnummer: WO 2006/008030

(56) Entgegenhaltungen:
- SUKSAMRARN S ET AL: "Antimycobacterial activity of prenylated xanthoones from the fruits of Garcinia mangostana" CHEMICAL AND PHARMACEUTICAL BULLETIN, Bd. 51, Nr. 7, Juli 2003 (2003-07), Seiten 857-859, XP002345097
- YATES P ET AL: "The structure of mangostin" JOURNAL OF THE AMERICAN CHEMICAL SOCIETY, Bd. 80, Nr. 7, 5. April 1958 (1958-04-05), Seiten 1691-1700, XP002345098 in der Anmeldung erwähnt
- CHEN S-X ET AL: "Active constituents against HIV-1 protease from Garcinia mangostana" PLANTA MEDICA, Bd. 62, Nr. 4, 1996, Seiten 381-382, XP008052625 in der Anmeldung erwähnt

## Beschreibung

### HINTERGRUND DER ERFINDUNG

Die Erfindung betrifft ein Verfahren zur Gewinnung und Reinigung von reinem α-Mangostin aus der Schale der Frucht ("Mangostane") von Garcinia mangostana. Die Mangostane ist die Frucht des aus dem malaiischen Archipel stammenden, in den gesamten Tropen verbreiteten und bis zu 15 m hohen Baums Garcinia mangostana.

Die etwa mandarinen- bis orangengroßen, dunkelviolett bis bräunlich-purpur gefärbten Früchte werden aufgrund des intensiv süß-säuerlichen Aromas ihres Fruchtfleisches sehr geschätzt. Im Saft und in der 8 mm dicken, festen, lederartigen Fruchthülle wurden unter anderem die gelbfarbenen Xanthon-Derivate α-Mangostin und γ-Mangostin nachgewiesen. α-Mangostin ist 1,3,6-Trihydroxy-7-methoxy-2,8-bis-(3-methyl-but-2-enyl)-xanthen-9-on der Formel 1, γ-Mangostin der in 7-Position freie Alkohol davon.

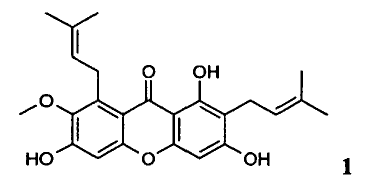

In der Literatur ist α-Mangostin als Antihistamin- und Antiserotonin-wirksame Substanzen beschrieben. Weiterhin wird es heutzutage von der kosmetischen Industrie eingesetzt. Demzufolge besteht ein großer Bedarf an der reinen Verbindung.

In der Literatur finden sich verschiedene Vorschläge für Methoden zur Reinigung und Isolierung von α-Mangostin. So wurde bereits 1958 von Yates et al. (J. Am. Chem. Soc. (1958) 80,1691) eine Extraktion der Fruchtschalen zur Gewinnung von Mangostin von Garcinia mangostana offenbart. Bei diesem Verfahren war jedoch, wie bei allen späteren Publikationen, nach der Extraktion des Pflanzenmaterials eine chromatographische Trennung zur Isolierung des α-Mangostins notwendig. (Sakai et al.Chem. Pharm. Bull (1993) 41, 958; Govindachari et al. Tetrahedron (1971), 27, 3919; Gopalakrishnan et al. J. Nat. Prod. (1997) 60, 519, Chen et al. Planta Med. (1996) 62, 381; Iinuma et al. J. Pharm. Pharmacol. (1996) 48, 86 1)

Der gestiegene Bedarf an α-Mangostin kann mit diesen Verfahren nicht abgedeckt werden. Aufgabe der vorliegenden Erfindung war es also ein einfaches und schnelles Verfahren zur Isolierung und Reinigung von α-Mangostin bereit zu stellen. Bevorzugt sollte dieses Verfahren großtechnisch durchführbar sein.

### DETAILLIERTE BESCHREIBUNG DER ERFINDUNG

Überraschenderweise wurde nun gefunden, dass hochreines α-Mangostin aus einem Extrakt gemahlener Mangostanen-Schalen erhalten werden kann, wenn man diesen Extrakt einer weiteren Verteilung zwischen Toluol und einem Diol unterzieht.

Der vorliegenden Erfindung liegt also ein Verfahren zur Gewinnung von α-Mangostin aus gemahlenen Mangostanen-Schalen zu Grunde, worin man nacheinander folgende Schritte durchführt:
a) Extraktion des Pflanzenmaterials mit einem Extraktionslösungsmittel, bestehend aus einem aromatischen Lösungsmittel;
b) Einengen und Kristallisieren aus einem aromatischen Lösungsmittel;
c) Lösen des Rohprodukt in einem Diol und gegebenenfalls Toluol;
d) Verteilung des gelösten Rohproduktes zwischen Toluol und einem Diol;
e) Einengen der aromatischen Phase und Umkristallisieren aus Alkohol/Wasser.

Bevorzugt wird das obige Verfahren, worin in Schritt a) das Extraktionslösungsmittel die Bedeutung von Toluol hat.

Bevorzugt wird das obige Verfahren, worin in Schritt a) die Extraktion bei 40-100°C, bevorzugt 50-90°C, besonders bevorzugt 59-71°C erfolgt.

Bevorzugt wird das obige Verfahren, worin in Schritt b) aus Toluol kristallisiert wird.

Bevorzugt wird das obige Verfahren, worin in Schritt c) zum Lösen ein Mischung aus einem Diol und Toluol in einem Verhältnis von 90:1 bis 99:1, bevorzugt 93:7 bis 98:2, besonders bevorzugt 95:5 bis 97:3, am meisten bevorzugt etwa 96:4, eingesetzt wird.

Bevorzugt wird das obige Verfahren, worin in Schritt c) das Diol die Bedeutung von 1,2-Ethandiol, 1,2-Propandiol, 1,3-Propandiol, 1,2-, 1,3- oder 1,4-Butandiol hat, besonders bevorzugt 1,2-Ethandiol.

Bevorzugt wird das obige Verfahren, worin in Schritt c) das Lösen des Rohprodukts bei 60-100°C, bevorzugt 70-90°, besonders bevorzugt 80°C erfolgt.

Bevorzugt wird das obige Verfahren, worin in Schritt d) die Verteilung in einer Kühni-Kolonne im Gegenstrom erfolgt.

Bevorzugt wird das obige Verfahren, worin in Schritt d) das Diol die Bedeutung von 1,2-Ethandiol, 1,2-Propandiol, 1,3-Propandiol, 1,2-, 1,3- oder 1,4-Butandiol hat, besonders bevorzugt 1,2-Ethandiol.

Bevorzugt wird das obige Verfahren, worin in Schritt d) die Verteilung bei 10-35°C, bevorzugt 15-30°, besonders bevorzugt 20-25°C erfolgt.

Bevorzugt wird das obige Verfahren, worin in Schritt e) das Umkristallisieren mit Ethanol/Wasser erfolgt.

Besonders bevorzugt ist das obige Verfahren, wenn das Pflanzenmaterial vor der Extraktion in Schritt a) für 5-20 Stunden, bevorzugt 10-15 Stunden, besonders bevorzugt 12-13 Stunden in Wasser vorgeweicht und anschließend mit der zwei- bis vierfachen, bevorzugt dreifachen Menge Toluol versetzt wird. Am meisten bevorzugt, wenn das Toluol eine Temperatur von 40-90°C, bevorzugt 50-80°C, besonders bevorzugt 60-70°C aufweist.

Somit ist besonders bevorzugt ein Verfahren zur Gewinnung von α-Mangostin aus gemahlenen Mangostanen-Schalen, worin man nacheinander folgende Schritte durchführt:
- Versetzen von in Wasser vorgeweichtem Pflanzenmaterial mit 40-90°C warmem Toluol;
- Extraktion bei 40-100°C mit einem Extraktionslösungsmittel, bestehend aus Toluol;
- Einengen und Kristallisieren aus Toluol;
- Lösen des Rohprodukt in einer Mischung aus 1,2-Ethandiol und Toluol in einem Verhältnis von 93:7 bis 98:2, bei 60-100°C;
- Verteilung des gelösten Rohproduktes zwischen Toluol und 1,2-Ethandiol bei 10-35°C;
- Einengen und Umkristallisieren aus Ethanol/Wasser.

Somit ist am meisten bevorzugt ein Verfahren zur Gewinnung von α-Mangostin aus gemahlenen Mangostanen-Schalen, worin man nacheinander folgende Schritte durchführt:
- Versetzen von in Wasser vorgeweichtem Pflanzenmaterial mit 60-70°C warmem Toluol;
- Extraktion bei 59-71 °C mit Toluol;
- Einengen und Umkristallisieren aus Toluol;
- Lösen des Rohprodukt in Mischung aus 1,2-Ethandiol und Toluol in einem Verhältnis von etwa 96:4, bei 80°C;
- Abkühlen auf RT und filtrieren der Lösung;
- Verteilung in einer Kühni-Kolonne mit Toluol bei 20-25°C im Gegenstrom;
- Einengen der Mischung zu einer rührfähigen Suspension;
- Abkühlen auf 10-15°C und absaugen;
- Umkristallisieren aus Ethanol/Wasser.

### VERWENDETE BEGRIFFE UND DEFINITIONEN

Unter einer " Kühni-Kolonne" wird im Rahmen der Erfindung eine Kolonne verstanden, bei der unter Rühren ein Lösungsmittel 1 von oben und gleichzeitig ein weiteres Lösungsmittel 2 von unten in die Kolonne eingetragen wird. Dabei weist das Lösungsmittel 2 eine geringere Dichte als das Lösungsmittel 1 auf. Sind die beiden Lösungsmittel nicht miteinander mischbar, so kann auf diesem Weg ein kontinuierliches Verteilungs-Verfahren realisiert werden. Dieser Typ von Kolonnen zur flüssig-flüssig Veteilung ist im Stand der Technik bekannt.

Unter einem "aromatischen Lösungsmittel" wird im Rahmen der vorliegenden Erfindung ein Lösungsmittel oder -gemische, ausgewählt aus der Gruppe, bestehend aus Benzol, Toluol, *o*-Xylol, *m*-Xylol, *p*-Xylol und Mesitylen verstanden.

Unter einem "Alkohol" wird im Rahmen der vorliegenden Erfindung eine verzweigte oder unverzweigte Alkylgruppen mit 1 bis 6 Kohlenstoffatomen und einer Hydroxylgruppe verstanden. Beispiele hierfür sind Methanol, Ethanol, Propanol, Propanol, Butanol, Pentanol, womit alle Isomere und Gemische davon umfasst sind.

Unter einem "Diol" wird im Rahmen der vorliegenden Erfindung eine verzweigte oder unverzweigte Alkylgruppen mit 2 bis 6 Kohlenstoffatomen und zwei Hydroxylgruppen verstanden. Beispiele hierfür sind Ethandiol, Propandiol, Butandiol, Pentandiol und Hexandiol womit alle Isomere und Gemische davon umfasst sind.

### BEISPIELE

Die nachfolgenden Beispiele dienen der Illustration des exemplarisch durchgeführten Verfahrens zur Gewinnung des α-Mangostins. Sie sind lediglich als mögliche, exemplarisch dargestellte Vorgehensweisen zu verstehen, ohne die Erfindung auf ihren Inhalt zu beschränken.

### EXTRAKTION

600 g gemahlene Mangostanen-Schalen werden mit 300 g Wasser versetzt, gemischt und über Nacht stehen gelassen. Das angemaischte Mahlgut wird anschließend in den Extrakteur gefüllt und 1000 ml warmes Toluol (60-70°C) zugegeben. Die Extraktion wird mit 8260 ml Toluol und einer Durchflussmenge von 2360-2700 ml/h bei 59-71 °C durchgeführt. Die entstandene Micella wird bei 70°C und 85-100 mbar eingedampft.

Es bleibt ein Rückstand: 56,7g, dunkelbraunes zähes Öl. Zum Rückstand werden 85 ml Toluol zugegeben und der Inhalt bei 70°C in Lösung gebracht. Nach Animpfen wird unter Rühren auf RT abgekühlt und weitere 15 ml Toluol zugegeben und bis auf 10°C abgekühlt. Die entstandene Suspension wird über einen Filter abgesaugt und mit 45 ml kaltem Toluol nachgewaschen. Das isolierten Rohprodukt 1 wird im Vakuum bei 55°C getrocknet; Ausbeute: 29,0 g.

### VERTEILUNG

1 kg Rohprodukt 1 wird in 12,5 11,2-Ethandiol und 522 ml Toluol bei 80°C gelöst und dann auf RT abgekühlt. Die Lösung wird filtriert und in einer Pilot-Kühni-Kolonne mit Toluol bei 20-25°C im Gegenstrom extrahiert. Hierbei wird die Rohproduktlösung am oberen, und Toluol am unteren Rand der Durchmischungszone unter Rühren zudosiert. (siehe Tabelle 1). Der aus der Verteilung erhalteneToluol-Extrakt wird i.V. bis zu einer noch rührfähigen Suspension eingeengt: 2653 g gelbe Suspension. Die Suspension wird auf 10-15°C abgekühlt und über einen Filter abgesaugt. Der Filterrückstand wird mit 450 ml kaltem Toluol nachgewaschen, trockengesaugt und i.V. bei 55°C getrocknet: Ausbeute Rohprodukt 2: 526,74 g gelbe Kristalle.

**Tabelle 1: Pilot-Kühni-Kolonne-Veteilung**

| | | **Rohprodukt - Lösung** | | **Toluol** | |
|---|---|---|---|---|---|
| **Zeit [min]** | **Rührer Stufe** | **Durchfluss [ml/min]** | **Gesamt- Durchsatz [I]** | **Durchfluss [ml/min]** | **Gesamt-Durchsatz [1]** |
| 0 | 8 | | | 485 | 1.00 |
| 5 | 8 | 54.7 | 0.30 | 511 | 4.31 |
| 20 | 6 | 44.0 | 1.12 | 522 | 12.90 |
| 35 | 6.5 | 45.0 | 1.72 | 524 | 19.35 |
| 45 | 5.75 | 46.0 | 2.38 | 522 | 26.76 |
| 65 | 6 | 49.0 | 3.20 | 515 | 35.04 |
| 75 | 5.5 | 58.0 | 3.90 | 614 | 42.40 |
| 105 | 5.5 | 63.0 | 5.67 | 616 | 60.00 |
| 163 | 5.5 | 59.0 | 9.20 | 610 | 95.00 |
| 183 | 5.5 | 76.0 | 10.10 | 766 | 105.00 |
| 210 | 5.5 | 76.0 | 12.50 | 767 | 129.00 |
| 223 | 5.5 | 76.0 | 13.40 | 768 | 138.70 |
| 251 | 5.5 | | 15.70 | 850 | 158.40 |
| 275 | 5.5 | | 17.14 | 850 | 175.20 |

### UMKRISTALLISATION

500 g Rohprodukt 2 werden in 1400 ml Ethanol bei 30-35°C gelöst, filtriert und der Filter mit 50 ml Ethanol nachgewaschen. Zur Lösung werden bei 30 - 35°C innerhalb von 25 Min. 725 ml Wasser unter Rühren zugefügt. und mit reinem α-Mangostin angeimpft. Die entstehende Suspension wird 90 Min. bei 27 - 30°C weiterrühren gelassen. Danach werden bei 27 - 28°C innerhalb von 40 Min 725 ml Wasser unter Rühren zugefügt und innerhalb von 35 Min. bis auf 10°C abgekühlt. Die Suspension wird über einen Filter abgesaugt, das Nutschengut mit 400ml kaltem Ethanol-Wasser-Gemisch (1:1) gewaschen. und i.V. bei 55°C getrocknet; Ausbeute α-Mangostin: 487.1g gelbe Kristalle.

## Patentansprüche

1. Verfahren zur Gewinnung von α-Mangostin aus gemahlenen Mangostanen-Schalen, worin man nacheinander folgende Schritte durchführt:
a) Extraktion des Pflanzenmaterials mit einem Extraktionslösungsmittel, bestehend aus einem aromatischen Lösungsmittel;
b) Einengen und Kristallisieren aus einem aromatischen Lösungsmittel;
c) Lösen des Rohprodukt in einem Diol und gegebenenfalls Toluol;
d) Verteilung des gelösten Rohproduktes zwischen Toluol und einem Diol;
e) Einengen der aromatischen Phase und Umkristallisieren aus Alkohol/Wasser,
wobei unter einem "aromatischen Lösungsmittel" ein Lösungsmittel oder -gemische, ausgewählt aus der Gruppe bestehend aus Benzol, Toluol, *o*-Xylol, *m*-Xylol, *p*-Xylol und Mesitylen, unter einem "Diol" eine verzweigte oder unverzweigte Alkylgruppe mit 2 bis 6 Kohlenstoffatomen und zwei Hydroxylgruppen und unter einem "Alkohol" eine verzweigte oder unverzweigte Alkylgruppe mit 1 bis 6 Kohlenstoffatomen und einer Hydroxylgruppe verstanden wird.

2. Verfahren nach Anspruch 1, worin in Schritt a) das Extraktionslösungsmmittel die Bedeutung von Toluol hat.

3. Verfahren nach den Ansprüchen 1 oder 2, worin in Schritt a) die Extraktion bei 40-100°C erfolgt.

4. Verfahren nach einem der Ansprüche 1-3, worin in Schritt b) aus Toluol kristallisiert wird.

5. Verfahren nach einem der Ansprüche 1-4, worin in Schritt c) zum Lösen eine Mischung aus einem Diol und Toluol in einem Verhältnis von 90:1 bis 99:1 eingesetzt wird.

6. Verfahren nach einem der Ansprüche 1-5, worin in Schritt c) das Lösen des Rohprodukts bei 60-100°C erfolgt.

7. Verfahren nach einem der Ansprüche 1-6, worin in Schritt d) die Verteilung in einer Kühni-Kolonne im Gegenstrom erfolgt.

8. Verfahren nach einem der Ansprüche 1-7, worin in Schritt d) die Verteilung bei 10-35°C erfolgt.

9. Verfahren nach einem der Ansprüche 1-5, worin in den Schritten c) und d) das Diol die Bedeutung von 1,2-Ethandiol hat.

10. Verfahren nach einem der Ansprüche 1-9, worin in Schritt e) das Umkristallisieren mit Ethanol/Wasser erfolgt.

11. Verfahren nach einem der Ansprüche 1-10, worin das Pflanzenmaterial vor dem Schritt a) für 5-20 Stunden in Wasser vorgeweicht und anschließend mit der dreifachen Menge an 40-90°C warmem Toluol versetzt wird.

## Claims

1. Process for obtaining α-mangostin from ground mangosteen rinds, in which the following steps are carried out one after the other:
a) extracting the plant material with an extraction solvent consisting of an aromatic solvent;
b) concentrating and crystallising from an aromatic solvent;
c) dissolving the crude product in a diol and optionally toluene;
d) partitioning the dissolved crude product between toluene and a diol;
e) concentrating the aromatic phase and recrystallising from alcohol/water, wherein by an "aromatic solvent" is meant a solvent or mixture of solvents selected from among benzene, toluene, *o*-xylene, *m*-xylene, *p*-xylene and mesitylene, by a "diol" is meant a branched or unbranched alkyl group having 2 to 6 carbon atoms and two hydroxyl groups and by an "alcohol" is meant a branched or unbranched alkyl group having 1 to 6 carbon atoms and one hydroxyl group.

2. Process according to claim 1, wherein in step a) the extraction solvent is toluene.

3. Process according to claim 1 or 2, wherein in step a) the extraction is carried out at 40-100°C.

4. Process according to one of claims 1-3, wherein in step b) crystallisation is carried out from toluene.

5. Process according to one of claims 1-4, wherein in step c) a mixture of a diol and toluene in a ratio of 90:1 to 99:1 is used for the dissolving.

6. Process according to one of claims 1-5, wherein in step c) the dissolving of the crude product is carried out at 60-100°C.

7. Process according to one of claims 1-6, wherein in step d) the partitioning is carried out in counter-current in a Kühni column.

8. Process according to one of claims 1-7, wherein in step d) the partitioning is carried out at 10-35°C.

9. Process according to one of claims 1-5, wherein in steps c) and d) the diol is 1,2-ethanediol.

10. Process according to one of claims 1-9, wherein in step e) the recrystallisation is carried out with ethanol/water.

11. Process according to one of claims 1-10, wherein the plant material is presoftened in water for 5-20 hours before step a) and is then combined with three times the amount of toluene warmed to 40-90°C.

## Revendications

1. Procédé d'obtention d'α-mangoustine à partir d'écorces de mangoustans broyées, où on accomplit successivement les étapes suivantes :
a) extraction du matériel végétal avec un solvant d'extraction consistant en un solvant aromatique;
b) concentration et cristallisation dans un solvant aromatique;
c) dissolution du produit brut dans un diol et éventuellement le toluène;
d) partage du produit brut dissous entre le toluène et un diol ;
e) concentration de la phase aromatique et recristallisation dans un alcool/l'eau,
où par « solvant aromatique » on entend un solvant ou des mélanges de solvants choisis dans le groupe consistant en le benzène, le toluène, le *o*-xylène, le *m*-xylène, le *p*-xylène et le mésitylène, par « diol » on entend un groupe alkyle ramifié ou non ramifié ayant 2 à 6 atomes de carbone et deux groupes hydroxyle et par « alcool » on entend un groupe alkyle ramifié ou non ramifié ayant 1 à 6 atomes de carbone et un groupe hydroxyle.

2. Procédé selon la revendication 1 où, dans l'étape a), le solvant d'extraction a la signification du toluène.

3. Procédé selon les revendications 1 ou 2 où, dans l'étape a), l'extraction a lieu à 40-100°C.

4. Procédé selon l'une des revendications 1-3 où, dans l'étape b), on cristallise dans le toluène.

5. Procédé selon l'une des revendications 1-4 où, dans l'étape c), on utilise pour la dissolution un mélange d'un diol et de toluène dans un rapport de 90: 1 à 99: 1.

6. Procédé selon l'une des revendications 1-5 où, dans l'étape c), la dissolution du produit brut a lieu à 60-100°C.

7. Procédé selon l'une des revendications 1-6 où, dans l'étape d), le partage a lieu dans une colonne de Kühni à contre-courant.

8. Procédé selon l'une des revendications 1-7 où, dans l'étape d), le partage a lieu à 10-35°C.

9. Procédé selon l'une des revendications 1-5 où, dans les étapes c) et d), le diol a la signification du 1,2-éthanediol.

10. Procédé selon l'une des revendications 1-9 où, dans l'étape e), la recristallisation a lieu avec l'éthanol/eau.

11. Procédé selon l'une des revendications 1-10 où, avant l'étape a), le matériel végétal est prétrempé dans l'eau pendant 5-20 heures puis additionné de trois fois la quantité de toluène chaud à 40-90°C.
